# EUROPÄISCHE PATENTSCHRIFT

(11) **EP 2 475 766 B1**
(45) Veröffentlichungstag und Bekanntmachung des Hinweises auf die Patenterteilung: **18.01.2017**
(21) Anmeldenummer: 10751816.9
(22) Anmeldetag: 08.09.2010
(51) Int. Cl.: C12N 5/071, G01N 33/50, A61K 35/60

(54) **SPONTAN KONTRAHIERENDE FISCHZELLAGGREGATE, DEREN VERWENDUNG UND VERFAHREN ZU DEREN ERZEUGUNG**
SPONTAINEOUSLY CONTRACTING CELL AGGREGATES DERIVED FROM FISH, THEIR UTILIZATION AND METHOD OF GENERATION
AGGREGATS DE CELLULES DE POISSON SE CONTRACTANT SPONTANEMENT, LEUR UTILISATION ET PROCEDE PERMETTANT LEUR GENERATION

(30) Priorität: 11.09.2009 DE 102009041254
(43) Veröffentlichungstag der Anmeldung: 18.07.2012
(73) Patentinhaber: Fraunhofer-Gesellschaft zur Förderung der angewandten Forschung e.V., 80686 München (DE)
(72) Erfinder: KRUSE, Charli, 23923 Herrnburg (DE); GRUNOW, Bianka, 17493 Greifswald (DE); RAPOPORT, Daniel, 23564 Lübeck (DE); CIBA, Philipp, 23568 Lübeck (DE)
(74) Vertreter: Katzameyer, Michael
(86) Internationale Anmeldenummer: PCT/EP2010/005509
(87) Internationale Veröffentlichungsnummer: WO 2011/029584

(56) Entgegenhaltungen:
- WO-A1-2005/108598
- DRIEVER WOLFGANG ET AL: "Characterization of a cell line derived from zebrafish (Brachydanio rerio) embryos" IN VITRO CELLULAR AND DEVELOPMENTAL BIOLOGY, THE ASSOCIATION, GAITHERSBURG, MD, US, Bd. 29A, Nr. 9, 1. September 1993 (1993-09-01), Seiten 749-754, XP009139697 ISSN: 0883-8364
- TYTGAT JAN: "How to isolate cardiac myocytes" CARDIOVASCULAR RESEARCH, Bd. 28, Nr. 2, 1994, Seiten 280-283, XP9139734 ISSN: 0008-6363
- BRETTE F ET AL: "Characterization of isolated ventricular myocytes from adult zebrafish (Danio rerio)" BIOCHEMICAL AND BIOPHYSICAL RESEARCH COMMUNICATIONS, ACADEMIC PRESS INC. ORLANDO, FL, US LNKD- DOI:10.1016/J.BBRC.2008.06.109, Bd. 374, Nr. 1, 12. September 2008 (2008-09-12), Seiten 143-146, XP023182677 ISSN: 0006-291X [gefunden am 2008-07-09]
- GOLDSTEIN J P ET AL: "Are Primary Cultures of Trout (Oncorhynchus mykiss) Ventricular Cardiomyocytes Metabolically Viable?" FISH PHYSIOLOGY AND BIOCHEMISTRY, KLUWER ACADEMIC PUBLISHERS, DO LNKD- DOI:10.1007/S10695-005-2492-2, Bd. 30, Nr. 2, 1. Juni 2004 (2004-06-01), Seiten 109-117, XP019241569 ISSN: 1573-5168
- NURMI ANTTI ET AL: "Electrophysiological properties of rainbow trout cardiac myocytes in serum-free primary culture" AMERICAN JOURNAL OF PHYSIOLOGY: REGULATORY, INTEGRATIVE AND COMPARATIVE PHYSIOLOGY, AMERICAN PHYSIOLOGICAL SOCIETY, US, Bd. 282, Nr. 4, 1. April 2002 (2002-04-01) , Seiten R1200-R1209, XP009139591 ISSN: 0363-6119
- KIMMEL C B ET AL: "STAGES OF EMBRYONIC DEVELOPMENT OF THE ZEBRAFISH" DEVELOPMENTAL DYNAMICS, WILEY-LISS, INC., NEW YORK, NY, US, Bd. 203, 1. Januar 1995 (1995-01-01), Seiten 253-310, XP008047852 ISSN: 1058-8388
- SINGH I S BRIGHT ET AL: "Development of primary cell cultures from kidney of fresh water fish Heteropneustes fossilis" INDIAN JOURNAL OF EXPERIMENTAL BIOLOGY, COUNCIL OF SCIENTIFIC & INDUSTRIAL RESEARCH, IN, Bd. 33, Nr. 8, 1. Januar 1995 (1995-01-01) , Seiten 595-599, XP009139698 ISSN: 0019-5189
- KUMAR G SUNIL ET AL: "Efficacy of fish and prawn muscle extracts as supplements of medium in development of a primary cell culture system from larval tissue of aquarium fish Poecilia reticulata" INDIAN JOURNAL OF EXPERIMENTAL BIOLOGY, COUNCIL OF SCIENTIFIC & INDUSTRIAL RESEARCH, IN, Bd. 36, Nr. 1, 1. Januar 1998 (1998-01-01) , Seiten 91-94, XP009139536 ISSN: 0019-5189
- GRUNOW B ET AL: "In vitro expansion of autonomously contracting, cardiomyogenic structures from rainbow trout Oncorhynchus mykiss" JOURNAL OF FISH BIOLOGY, ELSEVIER, NL LNKD- DOI:10.1111/J.1095-8649.2009.02535.X, Bd. 76, Nr. 2, 1. Februar 2010 (2010-02-01), Seiten 427-434, XP009139732 ISSN: 0022-1112 [gefunden am 2010-02-15]
- Bianka Grunow ET AL: "In vitro Developed Spontaneously Contracting Cardiomyocytes from Rainbow Trout as a Model System for Human Heart Research", Cellular Physiology and Biochemistry, 11 February 2011 (2011-02-11), pages 1-12, XP055189177, Basel, Retrieved from the Internet: URL:http://www.karger.com/Article/Pdf/3252 12 [retrieved on 2015-05-13]
- GRUNOW B ET AL: "In vitro expansion of autonomously contracting, cardiomyogenic structures from rainbow trout Oncorhynchus mykiss", JOURNAL OF FISH BIOLOGY, ELSEVIER, NL, vol. 76, no. 2, 1 February 2010 (2010-02-01), pages 427-434, XP009139732, ISSN: 0022-1112, DOI: 10.1111/J.1095-8649.2009.02535.X [retrieved on 2010-02-15]

## Beschreibung

Die Erfindung betrifft ein *in-vitro*-Verfahren zur Erzeugung von spontan kontrahierenden Fischzellaggregaten, die damit erhältlichen Fischzellaggregate und deren Verwendung, insbesondere zum Testen von biologischen Wirkstoffen und Pharmazeutika.

Herz-Kreislauferkrankungen sind eine der häufigsten Todesursachen und eine erfolgreichere Behandlung und Prävention dieser Erkrankungen ist das Ziel erheblicher Investitionen und Forschungsanstrengungen. Neben verbesserten Implantations- und Transplantationsmethoden, auch unter Verwendung neuer synthetischer oder teilsynthetischer Organe oder Organkomponenten, wie Blutgefäße, Bypässe, Herzklappen etc., besteht der Hauptansatz in der Entwicklung neuer Pharmazeutika, welche direkt oder indirekt eine Auswirkung auf die Herztätigkeit haben und beispielsweise speziell die Herzmuskelzellen oder andere Herzzellen beeinflussen. Zum Testen solcher Wirkstoffe werden herkömmlicher Weise Tierversuche vorgenommen.

Sowohl aus ethischen Gründen wie auch aus Kostengründen ist man jedoch bestrebt, den Umfang von Tierversuchen bei pharmazeutischen Tests möglichst niedrig zu halten, und mindestens die erste Testphase *in vitro* mit Zellen oder Zellkulturen durchzuführen.

Für diesen Zweck müssten bisher beispielsweise Zellkulturen von adulten humanen Herzzellen, insbesondere Kardiomyozyten, oder entsprechenden Zellen anderer Säugerspezies, z.B. Schwein, verwendet werden. Diese Zellen sind jedoch in der Regel nicht zu kultivieren oder verhalten sich nicht wie Zellen in einem natürlichen Gewebe (bilden z.B. keine kontrahierenden oder kontraktionsfähigen Zellaggregate bzw. dedifferenzieren in der Zellkultur). Andere nicht-humane Zellen weichen in ihrem Verhalten und ihren Eigenschaften von dem humaner Zellen ab. Beispielsweise weisen Kardiomyozyten von Maus oder Ratte andere Ionenkanäle als die entsprechenden humanen Zellen auf.

Vor diesem Hintergrund besteht die Aufgabe der Erfindung in der Bereitstellung von verbesserten Zellkulturen und *in-vitro-*Systemen zum Testen von biologischen Wirkstoffen und Pharmazeutika, die eine Auswirkung auf die Herztätigkeit allgemein und speziell auf Herzmuskelzellen oder andere Herzzellen haben, wobei die Zellkulturen und Testsysteme das Verhalten und die Eigenschaften von humanen Herzzellen in einem natürlichen Gewebe besser als bisherige *in vitro*-Systeme und sogar viele Tiermodelle widerspiegeln sollen.

Diese Aufgabe wird erfindungsgemäß gelöst durch das Verfahren zur Erzeugung der spontan kontrahierenden Zellaggregate nach Anspruch 1, die Materialzusammensetzungen und Zellkulturen, welche diese spontan kontrahierenden Zellaggregate enthalten, nach Anspruch 12 und 14 sowie die Verwendung dieser Zellaggregate nach Anspruch 18. Speziellere Ausführungsformen der Erfindung sind Gegenstand der weiteren Ansprüche.

Das erfindungsgemäße in *vitro*-Verfahren zur Erzeugung von spontan kontrahierenden Fischzellaggregaten nach Anspruch 1 umfasst die Schritte:
a) Mechanische Zerkleinerung und/oder partielle enzymatische Verdauung von Fischembryonen oder Fischlarven im Entwicklungsstadium von Anfang Augenpunktstadium bis Ende Dottersackstadium;
b) Überführung des zerkleinerten und/oder partiell verdauten Fischgewebes in enzymfreies Medium und Abzentrifugation des Überstands zur Gewinnung eines Zellpellets;
c) Resuspension des Zellpellets in Zellkulturmedium;
d) Kultivierung der Zellen unter mindestens einmaligem Mediumwechsel bis zur Bildung von spontan kontrahierenden Zellaggregaten.

Als Quelle für die erfindungsgemäß verwendeten Fischembryonen oder Fischlarven sind grundsätzlich beliebige Fischspezies geeignet, vorzugsweise solche der Osteichthyes, inbesondere Spezies der Teleostei. Spezielle Beispiele hierfür sind Spezies der Heringsfische (Clupeoidei), Lachsartigen (Salmonoidei), Karpfenfische (Cyprinidae), Aale (Anguillidae), Barsche (Percidae), Dorsche (Gadidae), Welse (Siluroidae), Plattfische (Pleuronectiformes), Hornhechte (Beloniformes), Störe (Acipenseriformes) etc.

Spezies der Lachsartigen z.B. Lachse, Forellen, insbesondere Bachforelle, Regenbogenforelle, Saiblinge, Huchen, Felchen, Renken, Äschen, Stinte, sind besonders bevorzugt.

Der Begriff "Fischembryos" bzw. "Fischlarven", wie hier verwendet, umfasst insbesondere Fischeier oder Fischlarven ab dem Augenpunktstadium (Ausbildung von Augenpunkten) bis zum Ende des Dottersackstadiums (Ernährung vom Dottersack).

Die partielle enzymatische Verdauung im erfindungsgemäßen Verfahren kann jede proteolytische Verdauung sein, die im Stand der Technik zum Aufschluss von tierischem Gewebe bekannt ist (z.B. mit Kollagenase, Chymotrypsin oder anderen Proteinasen) und umfasst oder besteht typischerweise aus einer Trypsin Behandlung, insbesondere Trypsin-EDTA-Behandlung.

Die Bedingungen der enzymatischen Behandlung werden so gewählt, dass es nach der Behandlung auf jeden Fall noch intakte Zellverbände gibt und nicht nur eine reine Einzelzellsuspension vorliegt.

Die für die jeweilige Art optimalen Bedingungen können vom Fachmann durch Routineversuche ermittelt werden. Typischerweise wird die Enzymbehandlung, z.B. Trypsin-EDTA-Behandlung, in einem Puffer mit 0.01 - 1 % Enzym, z.B. Trypsin, für einen Zeitraum von 15 Sekunden bis 10 Minuten, vorzugsweise 30 Sekunden bis 2 Minuten, durchgeführt.

Speziell im Falle einer Trypsinbehandlung ist es vorteilhaft (aber nicht unbedingt erforderlich), die Bedingungen so einzustellen, dass:
- das Volumen der sterilen Trypsinlösung etwa dem doppelten bis dreifachen des Gewebevolumens entspricht;
- die Gewebestückchen gleichmäßig mit der Lösung vermengt werden;
- der Verdau mit 37°C warmer Trypsinlösung bei 37°C durchgeführt wird;
- die Ansätze während des Verdaus wiederholt durchmischt werden;
- am Ende des Verdaus die Zerkleinerung des Gewebes durch Pipettieren verstärkt wird;
- am Ende des Verdaus die Proben durch kurze Lagerung auf Eis schnell wieder auf ihre Inkubationstemperatur gekühlt werden (4-25°C).

Neben der enzymatischen Behandlung wird vorzugsweise eine grobe mechanische Zerkleinerung, z.B. mit Hilfe einer Schere vorgenommen, wobei Gewebestücke in einem Größenbereich von typischerweise etwa 0,05-5 mm, beispielsweise 0,05-3 mm oder 1-5 mm, erzeugt werden. Auch eine stärkere Zerkleinerung ist jedoch schadlos möglich, und es ist sogar möglich, die enzymatische Behandlung ganz wegzulassen und den Gewebeaufschluss lediglich durch eine starke mechanische Zerkleinerung zu bewirken.

Zum Abbruch der enzymatischen Behandlung wird das zerkleinerte Gewebe in ein enzymfreies Medium überführt und der Überstand abzentrifugiert. Die Zentrifugation erfolgt mit einer geringen g-Zahl und soll lediglich ein Zellpellet vom flüssigen Überstand trennen. Typischerweise erfolgt die Zentrifugation bei 100 bis 180 g.

Die Kultivierung erfolgt in einem üblichen Zellkulturmedium, z.B. DMEM mit FKS, bei Temperaturen, die für die jeweiligen Fischzellen optimiert sind, typischerweise in einem Bereich von 4 bis 28°C, beispielsweise 4 bis 25°C, bevorzugt 15 bis 22°C. Vorzugsweise findet die Kultivierung in Gegenwart von CO₂, z.B. 0,5 bis 10 % CO₂, vorzugsweise 1 bis 5 % CO₂, statt.

Nach einigen Tagen Kultivierung, typischerweise 3 bis 7 Tagen, lassen sich mikroskopisch Areale mit spontan kontrahierenden Zellaggregaten beobachten.

Die leichte Kultivierbarkeit der Fischherzzellen und die spontane Bildung von kontrahierenden Zellaggregaten stellen einen immensen Vorteil gegenüber den Säugerzellkulturen des Standes der Technik dar.

Der Begriff "spontan kontrahierende Zellaggregate" wie hier verwendet, bedeutet, dass Kontraktionen (und Ausdehnungen) der Zellaggregate ohne äußere Stimulation, z.B. durch eine angelegte Spannung, erfolgen. "Spontan kontrahierende" Zellaggregate werden hier auch als "schlagende" oder "zuckende" Zellaggregate bezeichnet.

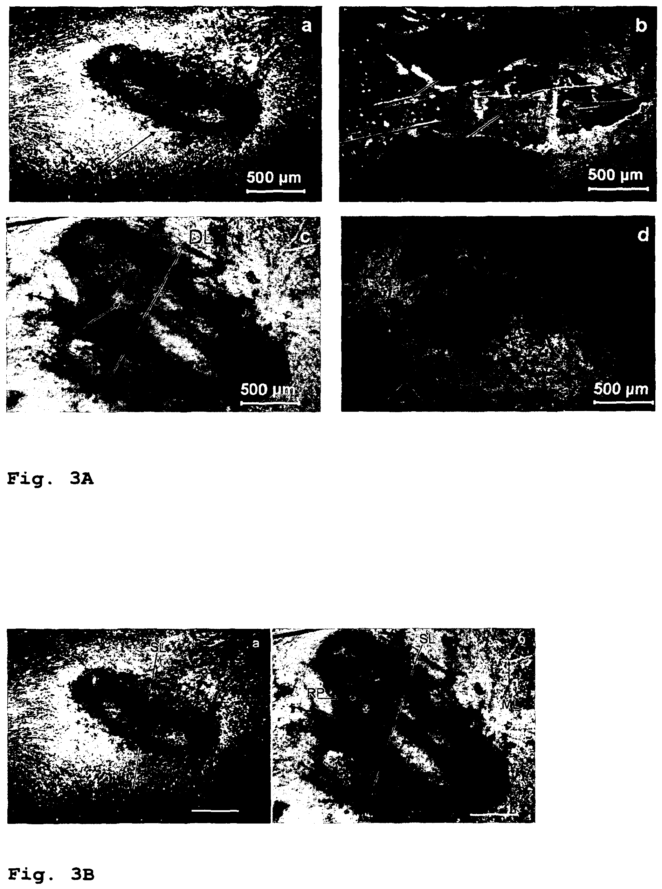

Die mit dem erfindungsgemäßen Verfahren erzeugten Zellaggregate und Zellkulturen können mehreren Passagen (beispielsweise mindestens 1 Passage) unterworfen werden. Dabei bleibt das Vermögen zur spontanen Kontraktion der Aggregate grundsätzlich erhalten, allerdings kann sich die Regelmäßigkeit und/oder die Kontraktionsfrequenz ("Schlagfrequenz") vorübergehend oder dauerhaft verändern (siehe Charakterisierungsversuche in den Beispielen). Dieser Effekt könnte dazu genützt werden, besonders vorteilhafte Kontraktionseigenschaften der Zellaggregate einzustellen. Auch die Gestalt der Zellaggregate ist variabel aber jeweils definiert, so wurden beispielsweise ballförmige und röhrenförmige Zellaggregate beobachtet (Fig. 3).

Ein weiterer Aspekt der Erfindung betrifft die mit dem oben beschriebenen erfindungsgemäßen Verfahren herstellbaren Fischzellaggregate mit dem Vermögen zur spontanen Kontraktion selbst sowie Materialzusammensetzungen und Zellkulturen, welche diese Zellaggregate enthalten.

Im Gegensatz zu natürlichen kontraktionsfähigen Zellaggregaten in Fischen sind die erfindungsgemäßen kontrahierenden Fischzellaggregate nicht Bestandteil eines Organismus oder eines in einem Organismus entstandenen Gewebes.

Die genannten Materialzusammensetzungen können ferner ein Träger- oder Hüllmaterial umfassen. Das Trägermaterial kann beispielsweise eine dreidimensionale Matrix bilden und aus natürlichen oder synthetischen Polymeren bestehen.

In vitro-Systeme, welche diese Zellaggregate oder Materialzusammensetzungen enthalten, bieten vorteilhafte Anwendungsmöglichkeiten in diversen Forschungsgebieten.

**Entwicklungsbiologie:** Studien zur Entwicklung eines Herzens mit Untersuchung an diversen Kanälen, Proteinen etc.

**Pathologie:** Anwendung von Ursache-Wirkungs-Komplexen in Bereich der Fischpathologie. So sterben aufgrund des z.B. Cardiomyopathy Syndrome (CMS) jährlich Millionen von Fischen. Bisher konnte in diesem Gebiet nur an Organismen geforscht werden. Mit den erfindungsgemäßen Systemen wäre es auch auf Zellebene möglich.

**Regenerative Medizin:** Durch die hohe Regeneration kann hier erstmals auch auf Zellebene die Regeneration von Fischherzzellen untersucht werden, bisher nur an lebenden Fischen (Zebrafische) möglich.

Untersuchungen der Erfinder haben bewiesen, dass Aggregate, die aus zwei verschiedenen Fischlarven stammen, zusammenwachsen können und die Kontraktion sich angleicht. Es wurde gezeigt, dass durch Zellverbindungsproteine aus ursprünglich zwei Aggregaten ein funktionierendes Synzytium hergestellt werden kann. Die Verwendung solcher Aggregate wäre sowohl in der Forschung als auch in der regenerativen Medizin von Interesse, da auf Zellverbandebene der Zusammenschluss zellfremder Gewebe untersucht werden könnte.Dieses Thema ist z.B. bei der Untersuchung von Abstoßungsreaktionen bei Transplantationen von großer Bedeutung.

**Pharmakologie:** Da die Herzzellaggregate in hoher Zahl generiert werden können, kann das *in vitro* System als Hochdurchsatz-Test-System verwendet werden. Ein weiterer Vorteil ist ihre spontane Kontraktion. Bisherige Modelle müssen meist elektrisch stimuliert werden, was die Ergebnisse verfälschen kann. Auch die Langzeit-Testung ist aufgrund von einer sehr langen Stabilität an den Aggregaten möglich. Weiterhin werden bisher hauptsächlich Mauszellen in der Pharmakologie für die Testung humaner Medikamente verwendet. Da die elektrophysiologischen Eigenschaften der Maus-Kardiomyozyten erheblich von humanen Kardiomyozyten abweichen, kommt es sehr häufig zu unterschiedlichen Reaktionen auf Medikamente. Die Fischaggregate weisen hingegen sehr starke Ähnlichkeiten zu humanen Kardiomyozyten auf, so dass dieselben Ionenkanäle im Fisch wie auch im Menschen auftreten. Da das erfindungsgemäße System zudem dreidimensional ist, könnten Medikamente erstmals an spontan kontrahierenden 3D-Zellverbänden in der Pharmakologie getestet werden.

Eine wichtige spezielle Anwendung dieser Materialzusammensetzungen oder Zellkulturen betrifft deren Verwendung zum Testen von biologischen Wirkstoffen (einschließlich Kosmetika) oder Pharmazeutika.

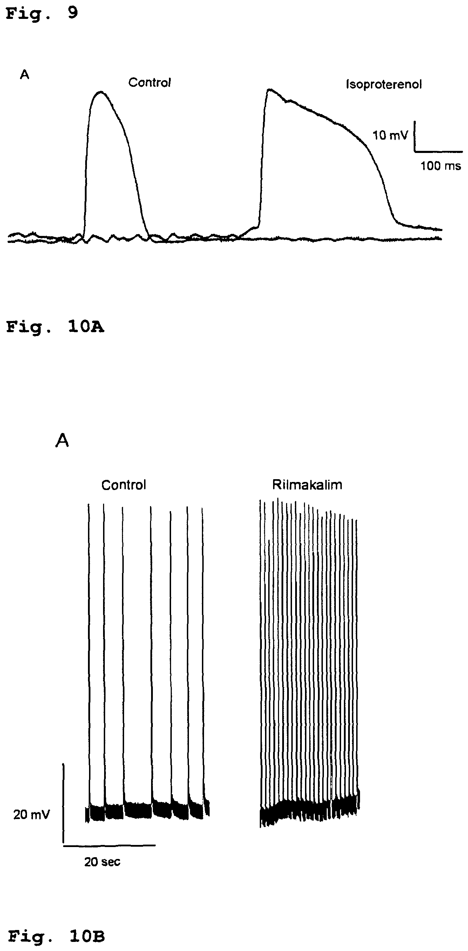

Vorzugsweise werden dabei biologische Wirkstoffe oder Pharmazeutika hinsichtlich ihrer Wirkung auf Herzmuskelzellen oder andere Herzzellen getestet. In Beispiel 5 und Fig. 10 sind beispielhafte Medikamententests beschrieben.

Die erfindungsgemäß bereitgestellten Materialzusammensetzungen und Zellkulturen mit spontan kontrahierenden Zellaggregaten sind für diese Anwendung besonders gut geeignet, da sie leicht, auch in großen Mengen, herstellbar sind und in vielen Eigenschaften humanen Zellen überraschenderweise sehr ähnlich sind, ähnlicher als z.B. Säugerzellen wie Maus, Ratte oder Schwein. Diese gilt insbesondere für die Kardiomyozyten, welche den Hauptbestandteil der kontraktionsfähigen Fischzellaggregate bilden, im Vergleich zu humanen Kardiomyozyten (siehe auch Brette et al. Biochem. (2008) Biophys. Res. Commun. 374, 143-146). Somit kann mit diesen Fischzellaggregaten erfindungsgemäß ein einfaches, aber leistungsfähiges in vitro-Modellsystem für humane Kardiomyozyten bzw. Herz(muskel)-gewebe zum Testen von biologischen Wirkstoffen oder Pharmazeutika hinsichtlich ihrer Wirkung auf Herzmuskelzellen oder andere Herzzellen bereitgestellt werden.

Eingehende Untersuchungen der Erfinder haben ergeben, dass die generierten Zellaggregate aus unterschiedlich alten Larven deutlich unterschiedliche Kontraktionen aufweisen. Die Kontraktionen wurden über drei Wochen für jedes Aggregat mittels Lichtmikroskopie und Videoaufnahme ermittelt. Das Larvenalter ist in Tagesgraden angegeben, da die Entwicklungszeit von Fischeiern/embryos und Fischlarven neben der Fischart stark von der Wassertemperatur abhängt. Ist das Wasser wärmer, schlüpfen die Larven schneller. Für die Maßeinheit "Tagesgrade" werden daher Temperatur und Tage miteinander multipliziert.

Grundsätzlich ist die Generierung von spontan kontrahierenden Aggregaten von Anfang "Augenpunktstadium" (210 Tagesgrade) bis Ende "Dottersackstadium" (500 Tagesgrade) möglich. Speziell hinsichtlich der Übereinstimmung mit humanen Herzzellen bzw. Herzzellaggregaten gibt es jedoch ein deutliches Optimum in einem bestimmten Entwicklungsstadium kurz vor und nach dem Schlupf der Fischlarven, spezieller bis etwa 1-50 Tagesgrade vorher oder nachher. Bei Regenbogenforellen liegt dieses Stadium in einem Bereich von etwa 320-400 Tagesgraden.

Wie aus den folgenden Tabellen ersichtlich, weisen von den Testaggregaten die Aggregate der Präparation 378 Tagesgrade (kurz nach dem Schlupf) die beste Übereinstimmung und die geringsten Abweichungen zur humanen Kontraktionsfrequenz auf. Die elektrophysiologischen Untersuchungen zeigten auch, dass das Ruhemembranpotential bei geschlüpften Larven dem Menschen näher ist (bei -60 mV), als bei Aggregaten von Larven im Ei (<-50mV).

| Larvenalter in Tagesgraden | Entwicklungsstand | Ratio der Generierung | Kontraktions-frequenz |
|---|---|---|---|
| 250 | Ei | 0,4 | 30 |
| 295 | Ei | 0,6 | 70 |
| 335 | Ei | 1,5 | 70 |
| 378 | Dottersack | 0,6 | 90 |
| 420 | Dottersack | 0,8 | 70 |
| 462 | Dottersack | 0,5 | 70 |

| Larvenalter in Tagesgraden | Median der Kontraktionsfrequenz | Minimum der Kontraktionsfrequenz | Maximum der Kontraktionsfrequenz |
|---|---|---|---|
| 252 | 25 | 22 | 37 |
| 294 | 65 | 42 | 75 |
| 335 | 60 | 30 | 83 |
| 378 | 90 | 85 | 92 |
| 420 | 62 | 45 | 81 |
| 426 | 70 | 43 | 90 |

Das Ratio (Ausbeute von Aggregaten) bei 378 Tagesgraden kann optimiert werden, da das Ratio davor und danach höher lag, so dass hier von einem unterschiedlichen Behandlung der Larven ausgegangen werden kann. So kann mit einer Verkürzung der Betäubungsinkubation die Bildung von Aggregaten vermehrt werden.

Die Verwendung von frisch geschlüpften Larven bietet ferner gegenüber der Isolierung aus dem Ei den Vorteil einer leichteren Handhabung, da die Larve nicht mehr aus dem Ei isoliert werden muss. Weiterhin sind die Aggregate mit zunehmenden Larvenalter größer, so dass elektrophysiologische Untersuchungen leichter durchgeführt werden können.

Es konnte gezeigt werden, dass die erfindungsgemäß erhaltenen Aggregate über einen Zeitraum von mehreren Wochen (bis zu 2 Monate) in ihrer Kontraktion stabil sind und somit sich als Langzeit-Test-System, z.B. in der Pharmakologie, wesentlich besser eignen als die derzeitigen kurzlebigen *in vitro* Kulturen.

Aufgrund der schnellen Generierung von Aggregaten kann dieses Test-System als Hochdurchsatz-Test-System verwendet werden, welches auf Zellverbandebene bisher nicht auf dem Markt existiert.

Ein wesentlicher Faktor hierbei ist die Zeit der Präparation. So konnte im Laufe der Arbeit das Protokoll so weit verbessert werden, dass 6 Larven binnen einer Viertelstunde präpariert werden können und somit innerhalb dieser Zeit ein 6-Well hergestellt werden kann. Da die Möglichkeit besteht, aus einer Larve drei bis maximal 5 Herzzellaggregate in der Passage 0 zu generieren, liegt die höchste anzunehmende Zahl bei 30 Kardiomyozytenaggregaten. Im Idealfall kann ein 96-Well durch die Präparation von 20 Fischlarven innerhalb von 50 Minuten Arbeitszeit besetzt werden.

Die erfindungsgemäß hergestellten Zellkulturen, welche neben den Kardiomyozyten und anderen Herzzellen auch noch weitere Zelltypen enthalten, können ferner auch zum Testen der Wirkung nicht-herzspezifischer biologischer Wirkstoffe, einschließlich Kosmetika und Pharmazeutika, auf diese Zelltypen vorteilhaft eingesetzt werden.

Die Durchführung aller dieser Tests kann mit beliebigen interessierenden Kandidatensubstanzen nach Standardverfahren erfolgen und die Bedingungen können vom Fachmann mit Routinetests unschwer optimiert werden.

Die folgenden nicht-beschränkenden Beispiele und Figuren sollen die vorliegende Erfindung näher erläutern.

### FIGURENBESCHREIBUNG

**Fig. 1** Prozentualer Anteil an Präparationen, in denen 1 (weiß), 2 (dunkelgrau) oder 3 (hellgrau) spontan schlagende Zellaggregate in Passage Null gebildet wurden. Jede Säule zeigt einen Präparationstag mit der Anzahl der präparierten Larven.
**Fig. 2** Schlagfrequenz eines Zellaggregates in Passage Null. Messung durch Videoaufnahmen am Mikroskop (Axiovert 40C; Zeiss, Deutschland) und anschließende optische Auszählung.
**Fig. 3A** Propagation eines schlagenden Zellaggregates in Passage 1 durch mechanische Teilung. (a) Aggregat vor der Teilung (Pfeil weist auf die Schnittstelle hin) und (b) beide Teile direkt nach der Separation (Pfeile). (c und d) Kontrahierende Elemente reorganisierten sich nach 7 Tagen, und bemerkenswerterweise formten sich zusätzlich kontrahierende Elemente de *novo* (Pfeil). DL ("Dividing Line") markiert die Stelle an der die Teile ursprünglich voneinander getrennt wurden. Aus den Zellaggregaten wachsen außerdem Zellen zu Monolayern aus (Pfeilkopf).
**Fig. 3B** Regeneration eines Zellaggregats
**Fig. 4** Immunochemische Analyse mit herzspezifischen Antikörpern. Die mittlere Spalte zeigt eine immunohistochemische Untersuchung auf Kryotomschnitten vom Herzgewebe einer adulten Regenbogenforelle. Als Vergleich wurden immunocytochemische Untersuchungen auf den kontrahierenden Zellaggregaten durchgeführt.
**Fig. 5** Elektronenmikroskopische Aufnahmen eines spontan kontrahierenden Zellaggregates. (a) eine ausgebildete Kardiomyozyte mit der typischen gestreiften Muskulatur mit Aktin- und Myosinfilamenten. (b) zeigt eine Zelle, die in der Entwicklung zur Herzzelle ist. Auffallend sind die Anfänge der gestreiften Muskulatur.
**Fig. 6** Stereomikroskopische Aufnahmen (Discovery. V8; Zeiss, Deutschland) einer Regenbogenforelle-Zellkultur. Bildung des Areals 1 7 Tage nach der Präparation; Areal 2 war bereits zu Beginn vorhanden und begann am 14. Tag spontan zu kontrahieren. Das Areal 3 wurde nach drei Wochen in Kultur neu gebildet.
**Fig. 7** PCR-Assay zum Nachweis von Zellverbindungsproteinen in den Zellaggregaten
**Fig. 8** Wachstum und Vereinigung von Zellaggregaten auf einem MEA
**Fig. 9** Aktionspotentialparameter der Zellaggregate im Vergleich zu Maus und Mensch (intrazelluläre Ableitung)
**Fig. 10** Wirkung von Herzmedikamenten auf die Zellaggregate; A: Isoproterenol (Calciumkanalagonist); B: Rilmakalim (Kaliumkanalblocker)
**Fig. 11** Regeneration von Zellaggregaten in Passage 0; a: vor Verletzung; b: direkt nach Verletzung; c: 2 Wochen nach Verletzung, Pfeile deuten auf regenerierte Zellen
**Fig. 12** A: Zellproliferation bei geteilten/verletzten Aggregaten; B: (a) Kontrolle; (b): geteiltes Aggregat
**Fig. 13** Longitudinalschnitt durch ein Herzzellaggregat (EM)
**Fig. 14** Entstehung einer Herzzelle (EM)

### BEISPIEL 1

### Präparation und Charakterisierung der Zellaggregate

**Verwendete Tiere:** Regenbogenforelle (*Oncorhynchus mykiss,* Walbaum) verschiedener Entwicklungsstadien von Eiern im Augenpunktstadium bis hin zu Larven am Ende des Dottersackstadiums wurden für die Versuche eingesetzt.

### Vorbereitungen:

1: Eier der Regenbogenforelle wurden in einer 0,01% Formaldehydlösung für 15 Minuten inkubiert und anschließend zwei Mal mit PBS (Dulbecco's Phosphate buffered saline, Gibco Invitrogen, Paisley U.K.) gewaschen. Anschließend wurde der Embryo aus seinem Ei mit einer Schere und Pinzette isoliert.
2: Larven wurden mit Tricaine-Methansulphonat (MS-222) (0,05mg/ml Aquarienwasser) betäubt.
   *Medium DMEM-20%FKS:*
   DMEM (Dulbecco's Modified Eagle Medium) 4,5 g/L Glukose (Gibco, Karlsruhe) wurde mit 1% Penicillin (10.000 Units/ml)/ Streptomycin (10 mg/ml) (PAA Laboratories, Österreich) und 20% Fetales Kälberserum (PAA Laboratories, Pasching, Österreich) versetzt.

Embryonen bzw. Larven wurden jeweils in 500 µl 0,1 % Trypsin/ EDTA gelöst in PBS (PAA Laboratories, Österreich) für 30 Sekunden bis 2 Minuten inkubiert und dabei zusätzlich mit einer Schere mechanisch zerkleinert. Der enzymatische Verdau wurde gestoppt, in dem das Gewebe mit einer 1000µl Pipette (Sarstedt, Deutschland) in 5ml des DMEM -FKS 20 %- Mediums überführt wurde. Anschließend wurde alles bei 130 rcf für 5 Minuten zentrifugiert. Das entstandene Pellet wurde in 2,5 ml Medium resuspendiert und die Zellsuspension jeder Larve in einen 6-Well-Träger (Techno Plastic Products AG (TPP) Trasadingen, Schweiz) gegeben. Die Zellen wurden bei 20°C und 1,9% CO₂ gehalten. Nach zwei Tagen wurde erstmals ein Mediumwechsel durchgeführt, wobei das vorhandene Medium und alle nicht angewachsenen Gewebestücke und Zellen mit abgenommen wurden und dieselbe Menge an frischem Medium hinzugegeben wurde. Im Falle von Verunreinigungen durch Bakterien oder Pilze wurden als Antibiotika Gentamycin (10mg/ml; Biochrom AG, Deutschland) und Kanamycin (5mg/ml; Biochrom AG, Deutschland.) und als Antimykotikum Amphotericin (250µg/ml, Biochrom AG, Deutschland) hinzugegeben.

### Ergebnisse:

### Bildung schlagender Zellaggregate

In der Zellkultur der präparierten Regenbogenforellenlarven konnten in der Regel 7 Tagen nach der Präparation via Mikroskop (Axiovert 40C; Zeiss, Deutschland) und Videoaufnahme schlagende Zellaggregate nachgewiesen werden. In seltenen Fällen bildeten sich diese bereits nach drei Tagen aus. Die Morphologie dieser Aggregate war zum Teil sehr verschieden. In Passage Null wiesen Sie in der Regel Ballform auf und schlugen sehr regelmäßig. In Passage 1 hingegen hatten sie eine röhrenförmige Struktur und zuckten zudem sehr unkontrolliert und an mehreren Stellen ein und desselben Gewebes. Die Passage 1 wurde durch die Trypsinierung der Zellkulturen erreicht. Dafür wurde das Medium der Kultur abgesaugt und die Zellen mit PBS gespült. Anschließend wurde die Ablösung der Zellen durch eine Inkubation mit 0,1 % Trypsin/EDTA für 1 min bei 37°C erreicht. Durch Zugabe des dreifachen Volumens an Mediums wurde der Verdau gestoppt und die Zellen bei 130 rcf für 5 min zentrifugiert. Die röhrenförmigen zuckenden Aggregate traten in den Kulturen ab den 10. Tag nach Passgierung auf, wobei es nicht erforderlich war, dass bereits in Passage Null ein schlagendes Aggregat vorhanden war.

### Wahrscheinlichkeit für die Bildung von schlagenden Aggregaten

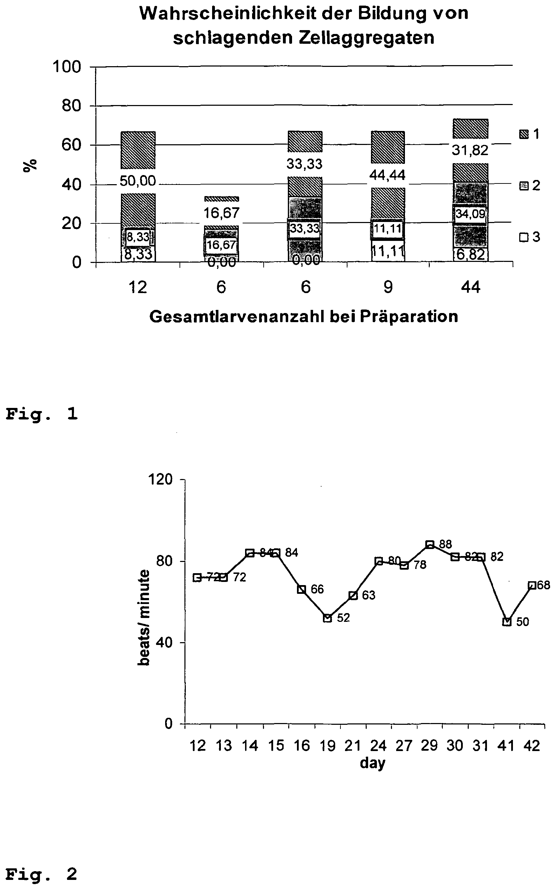

Für die Errechnung der Wahrscheinlichkeit für die Bildung von schlagenden Aggregaten wurden die Anzahl von präparierten Larven mit herausgebildeten schlagenden Aggregaten durch die Gesamtzahl der präparierten Larven dividiert. Die Präparationen zeigten, dass mit einer Wahrscheinlichkeit von über 70% spontan rhythmisch schlagende Zellaggregate gebildet werden konnten. Aus der Präparation einer einzelnen Larve konnten bis zu drei spontan kontrahierende Zellaggregate gebildet werden (Fig. 1).

### Schlagfrequenz

Weiterhin konnte gezeigt werden, dass die Aggregate über einen längeren Zeitpunkt eine Kontraktion aufwiesen, wobei während der Messung per Mikroskop (Axiovert 40C; Zeiss, Deutschland) und Videoaufnahme die Zellaggregate gleichmäßig schlugen. In Fig. 2 ist die Schlagfrequenz pro Minute eines spontan kontrahierenden Zellaggregates aus Passage Null einer präparierten Regenbogenforellenlarve über einen Zeitraum von 42 Tagen beispielhaft dargestellt. Die Abweichungen in den Frequenzen, die in diesem Fall zwischen 50 und 88 Schlägen pro Minuten lagen, können durch Stressfaktoren, wie z.B. verbrauchtes Medium oder lange Belichtung unter dem Mikroskop, hervorgerufen worden sein.

### Propagation und Regeneration der Zellaggregate

In Fig. 3A ist die Propagation eines Aggregates der Passage 1 abgebildet. Es konnte durch mechanische Teilung einzelner Aggregate mit Hilfe eines Skalpells und anschließender Überführung der beiden Teile in einzelne frische Wells gezeigt werden, dass die Aggregate vermehrbar sind. Nach einer Regeneration von einigen Tagen begannen beide Teile erneut schlagen. Trotz Trennung der Aggregate sind also beide Abschnitte weiterhin in der Lage zu kontrahieren. Der isolierte Bereich kann zudem regeneriert werden, so dass die ursprüngliche Form erneut erreicht wird und auch der neue Bereich eine Kontraktion aufweist. Eine mehrfache Propagation eines Aggregates ist daher nicht auszuschließen. Das Phänomen deutet auf mehrere Herzschrittmacherzellen und/oder die Generierung von Herzschrittmacherzellen hin.

Fig. 3B zeigt, dass nach Teilung der andere Bereich wieder neu gebildet wurde.

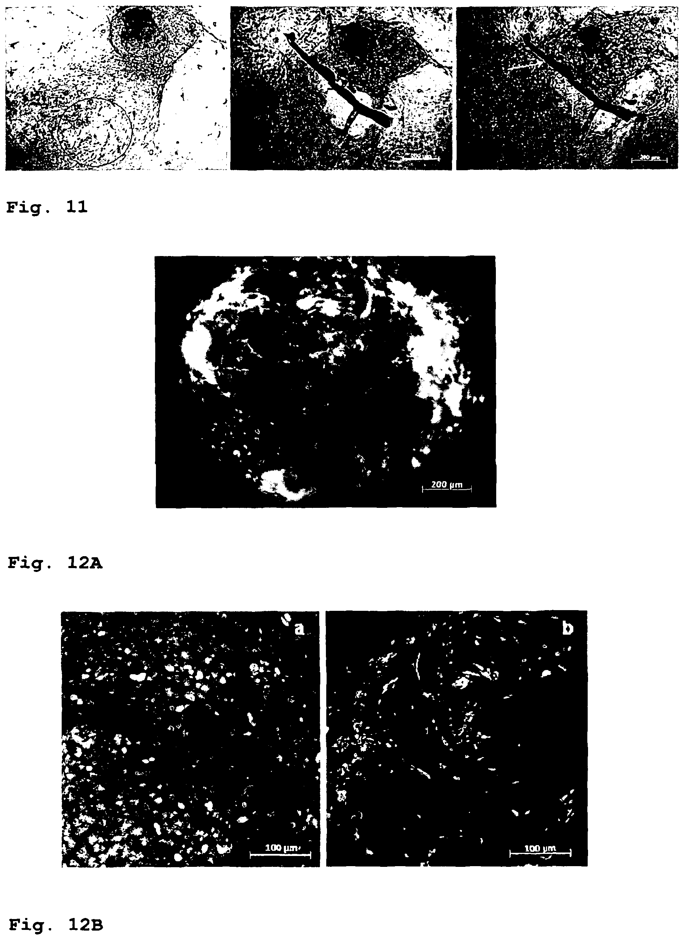

Das Zusammenwachsen von Zellen ist nach Trennung innerhalb eines Aggregates möglich. Fig. 11 beweist, dass die Kontraktion trotz Verletzung wieder einsetzt (rote Kreise) und sich dem der ursprünglichen Frequenz (vor der Verletzung) anpasst. Fig. 11 a: vor Verletzung; b: direkt nach Verletzung; c: 2 Wochen nach Verletzung, Pfeile deuten auf regenerierte Zellen

Um Zellteilung von Kardiomyozyten bei der Regeneration der Aggregate nachzuweisen, wurde der Proliferationsnachweis durch Einbau von EdU (grün) getestet. Das Aggregat wurde durch Hineinstechen in der Mitte des Aggregats verletzt. Dies ist in Fig. 12A in der Immunfärbung als schwarzer Bereich in der Mitte deutlich erkennbar. Man erkennt deutlich die Troponin I Strukturen (rot) als Herzzellnachweis um die Läsion. Direkt an dessen Rand und in dieses hinein findet man EdU positive Zellen, die aktiv proliferierende Zellen nachweisen. Blau sind Zellkerne, die sich nicht geteilt haben.

Als Kontrolle wurde zusätzlich ein nicht geteiltes Aggregat gefärbt. Von beiden Färbungen wurden mit LSM Aufnahmen in einer Ebene und ein z-Stapel gemacht. Als Ergebnisse zeigte sich (Fig. 12B), dass das ungeteilte Aggregat (a) weniger EdU positive Zellen aufweist (grün) und zudem diese zufällig über das gesamte Aggregat verteilt sind. Im Gegensatz dazu zeigte das geteilte Aggregat (b) ein hohes Aufkommen an EdU positiven Zellen im Bereich der Verletzung, so dass hier von einer hohen Zellproliferation ausgegangen werden kann.

### Untersuchung der Reaktion von Zellaggregaten auf die Zugabe von Kalium

In einem weiteren Versuch konnte die Reaktion eines Zellaggregates, welches bereits 16 Tage in Kultur kontrahierte, auf Kalium getestet werden. So wurde unter dem Lichtmikroskop (Axiovert 40C; Zeiss, Deutschland) mit Videoaufnahme gezeigt, das sich die Schlagfrequenz dieses Aggregates über einen Zeitraum von 10 Minuten von 66 Schläge pro Minute auf 72 Schläge pro Minute erhöhte. Nach diesen 10 Minuten wurde eine 1,75 mM Kaliumchlorid-Lösung hinzugeben. Die Kontraktion dieses Aggregates schlug nach Zugabe der KCl-Lösung 6 Sekunden in der dreifachen Geschwindigkeit wie zuvor und verlangsamte sich, bis es 10 Sekunden nach Zugabe stoppte und keine Kontraktionen mehr erkennbar waren. Nachdem das Medium mit der 1,75mM KCl-Lösung entfernt wurde und durch frisches DMEM-20% FKS ersetzt wurde, begann das Zellaggregat nach 2 Minuten erneut zu schlagen. Allerdings betrug die Schlagfrequenz anfangs nur 16 Schläge pro Minute, was sich in den folgenden drei Tagen zur ursprünglichen Frequenz regenerierte.

### BEISPIEL 2

### Identifizierung von Cardiomyocyten

### 1. Nachweis von herzspezifischen Proteinen mittels Immun-cytochemie und Immunhistochemie:

Der Nachweis durch die verwendeten Antikörper wurde zuerst auf Spezifität bei der Regenbogenforelle getestet. So wurde gezeigt, dass die folgenden Antikörper bei der Regenbogenforelle nur gegen jene Herzproteine gerichtet sind, wie sie auch beim Menschen und der Maus vorkommen.

### Vorbereitung für Immunhistochemie

Das Herz einer adulten Regenbogenforelle wurde für 30 Sekunden in Isopentan und anschließend eine Minute in flüssigem Stickstoff eingefroren, Das Organ wurde mit einem Kryostat in 12 µm dicke Gewebestücke geschnitten. Die Gewebeschnitte werden auf physikalisch oder chemisch aktivierte Deckgläser aufgebracht und kovalent auf der Glasoberfläche verankert. Die Deckgläser mit dem aufgebrachten Herzschnitten werden in millimeter-große Fragmente unterteilt (BioChips™, Euroimmun, Lübeck, Deutschland) und auf Testfelder von Objektträgern aufgebracht (BioChip Mosaics™, Euroimmun, Lübeck, Deutschland).

### Vorbereitung für Immuncytochemie

Ein Tag vor der Immunfärbung wurden die Zellaggregate auf die Chamber-Slides (BD Bioscience, Bredford, MA, USA) überführt, in dem sie aus dem ursprünglichen Well mit einem Skalpell vorsichtig ausgeschnitten und abgelöst wurden. Das Aggregat wurde durch vorsichtiges Aufnehmen mit einer Pipette überführt. Das Aggregat wurde über Nacht weiterhin bei 20 °C und 1,9 % CO2 in DMEM-20 % FKS kultiviert.

Die Vorbereitung für die Immunfärbung der Aggregate beinhaltete das Abnehmen des alten Mediums und zweimaliges Spülen mit PBS. Die Fixierung erfolgte mit 1 ml Methanol-Aceton (7:3, -20 °C) und 1 µl DAPI (4',6-Diamidino-2-phenylindol, Roche, Basel, Schweiz) pro Well. Nach einer fünfminütiger Inkubation und Kernfärbung bei Raumtemperatur erfolgte eine dreimalige Spülung mit PBS.

Bei den Präparaten für die Immunhisto- und Immuncytochemie wurden die unspezifischen Bindungen durch 16,5 µl Ziegen-Normalserum (Vector Laboratories, Burlingame, CA, USA) gelöst in 1ml PBS 20 Minuten bei Raumtemperatur abgeblockt.

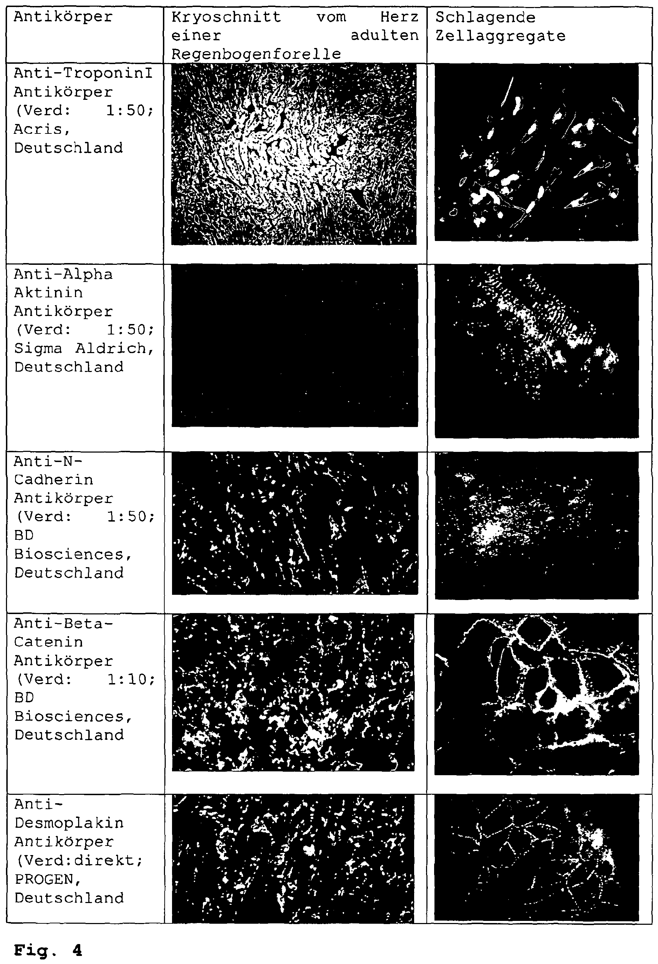

Nach dem Absaugen der Blocklösung wurde der spezifische Erstantikörper hinzugegeben. Die Verdünnung der primären Antikörper wurde mit TBST+ 0,1 % BSA hergestellt (siehe Abb. 4, Spalte 1). Die Inkubation in einer feuchten Kammer bei 37 °C betrug eine Stunde, worauf eine dreimalige Spülung mit PBS folgte. Anschließend wurde der Zweitantikörper Cy3-(conjugated AffiniPure Goat Anti-Maus IgG (High+Low); Verd.:1:400 mit PBS; Jackson Immunoresearch Laboratories Inc., USA) aufgetragen, der ebenfalls eine Stunde bei 37°C in einer feuchten Kammer inkubierte. Am Ende der Färbung wurde erneute drei Mal mit PBS gespült und anschließend das Präparat mit einem Deckglas und Vectashield (Vector Laboratories, Burlingame, CA, USA) konserviert. Die Auswertung erfolgte am Fluoreszenzmikroskop Axioskop 2 mot plus (Carl Zeiss Vision GmbH, Hamburg).

Die Immunfärbungen (Fig. 4) zeigten, dass die Antikörper an die für das Herz typischen Proteine auch bei den schlagenden Arealen gebunden haben. So hat der anti-TroponinI-Antikörper gegen das TroponinI und anti-alpha-Aktinin gegen die Proteine im Z-Band der Kardiomyozyte gebunden. Weiterhin konnte nachgewiesen werden, dass die Anti-N-Cadherin- und Anti-Beta-Catenin-Antikörper, welche Marker für Fascia adhaerens (Streifendesmosomen) sind, und der anti-Desmoplakin-Antikörper ebenfalls gegen die jeweiligen spezifischen Proteine gebunden haben.

### 2. Elektronenmikroskopie

Ein genauerer Nachweis für vorkommende Kardiomyozyten konnte mittels Elektronenmikroskopie geliefert werden. Für die Untersuchung wurden schlagende Zellaggregate aus präparierten Larven aus dem Augenpunktstadium und Larven, die 1-2 Tage zuvor geschlüpft waren, verwendet. Die schlagenden Aggregate, welche 3 Wochen alt waren, wurden mit einem Skalpell großflächig ausgeschnitten und abgelöst. Das Zellgewebe wurde in MONTI (2.5 % Glutaraldehyd in 0.1 M Cacodylat-Puffer) für 24 h fixiert und anschließend für eine Stunde in PBS gespült. Die Postfixierung erfolgte mit 1 % OsO₄ in 0.1 M Cacodylat-Puffer für 2 Stunden. Die Proben wurden im Folgenden mit Ethanol dehydriert und in Araldite (Fluka, Switzerland) eingebettet. Im Anschluss wurden ultradünne Schnitte in Uranylacetat und Bleicitrat (Ultrostainer Carlsberg System, LKB, Schweden) inkubiert. Am Ende erfolgte die Auswertung am Philips Elektronenmikroskop EM 400 bei 60 kV (Philips, Niederlande).

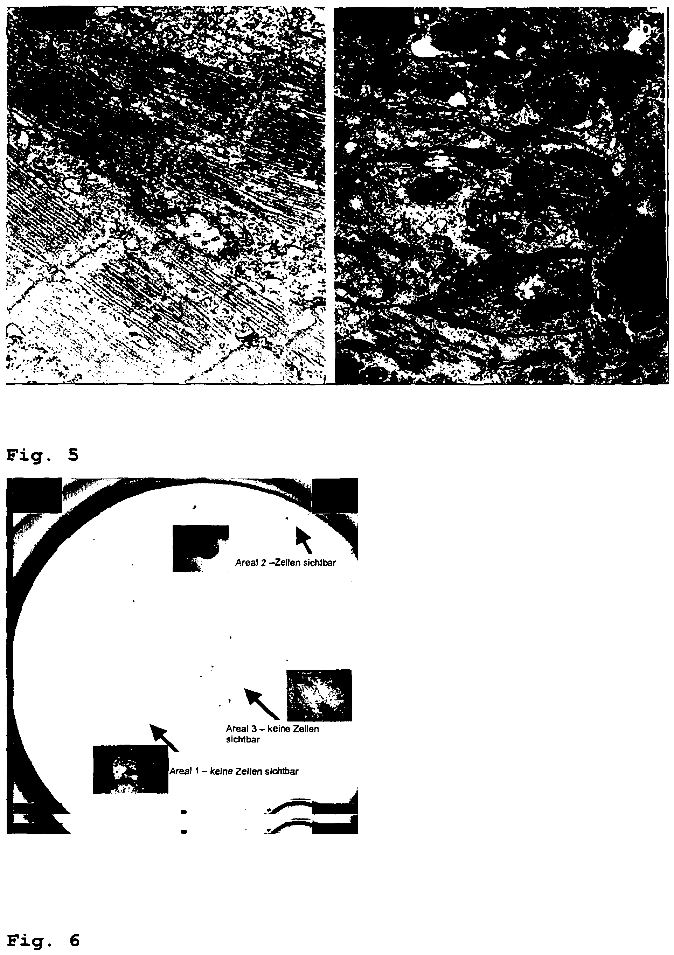

Die Elektronenmikroskopie zeigte, dass die schlagenden Zellaggregate typische gestreifte Herzmuskulatur mit Aktin- und Myosinfilamenten aufwiesen (Fig. 5a). Weiterhin wurde in den Arealen deutlich, dass neben den bereits vollständig entwickelten Kardiomyozyten auch Zellen vorhanden sind, die noch in der Differenzierung zur Herzzelle sind (Fig. 5b). Da die Differenzierung häufig Nachbarzellen von bereits ausgebildeten Kardiomyocyten betraf und zudem die Differenzierung mit den ersten Muskelfilamenten am Zellrand auftrat, könnte dies ein Indiz für eine Zell-Zell-Kommunikation sein.

### BEISPIEL 3

### Nachweis der Neubildung/Regeneration der Herzaggregate

### Stereomikroskopische Beobachtungen

Der Versuch über den Nachweis von Neubildung oder Regeneration der nachgewiesenen Cardiomyocytenaggregate erfolgte nach dem ersten Mediumwechsel, der zwei Tagen nach der Präparation stattfand. Von dem Well, in dem die Zellen/Gewebe der präparierten Larve kultiviert wurden, wurde eine Übersicht mit dem Stereomikroskop Discovery. V8 (Zeiss, Deutschland) und dem Programm Axiovision Rel 4.7 aufgenommen. Eine erneute Dokumentation erfolgte beim Auftreten einer spontanen Kontraktion eines Herzzellaggregates. Die Zellkulturen wurden jeweils am Montag, Mittwoch und Freitag auf kontrahierende Aggregate untersucht. In Fig. 6 ist die Übersicht einer Larvenzellkultur gezeigt, welche innerhalb von drei Wochen drei schlagende Herzaggregate bildete. Es konnte festgestellt werden, dass sich Aggregat 1, welches nach einer Woche auftrat, und Aggregat 3 (beginnende Kontraktion nach drei Wochen) neu gebildet haben. Im Gegensatz war Aggregat 2 (beginnende Kontraktion nach 2 Wochen) bereits bei der ersten Dokumentation vorhanden.

### BEISPIEL 4

### Charakterisierung der Zellaggregate als funktionierendes Synzytium/ Herzzellverband

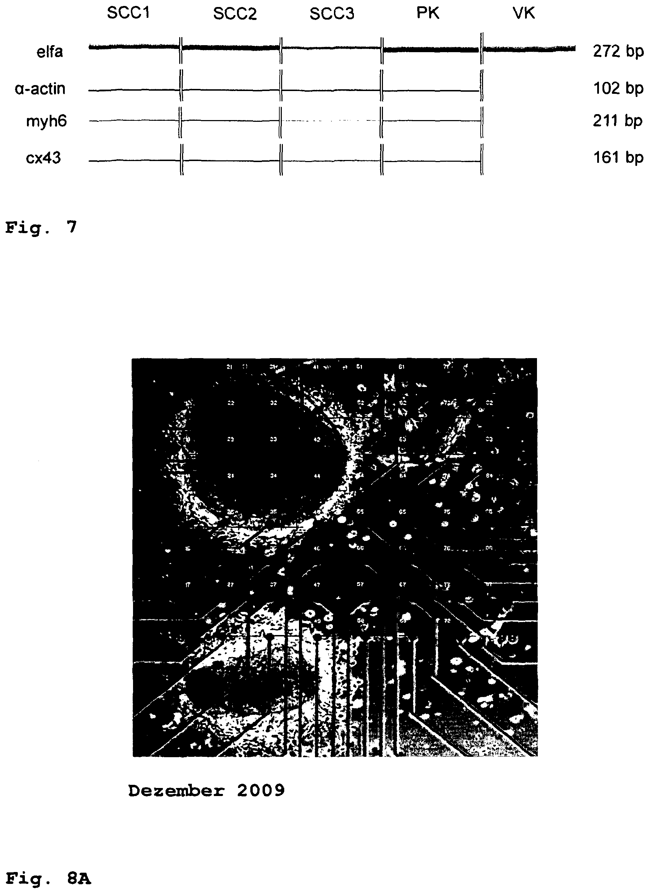

Neben den bereits erwähnten typischen Herzmuskelmarkern (alpha-Aktin, Aktinin, Myosin, TroponinI) wurden auch Nachweise über Zellverbindungsproteine bzw. *gap junction*/ *fascia adhaerens* erbracht. So konnten mittels Elektronenmikroskopie, PCR (Fig. 7) und Immunochemie in den kontrahierenden Zellaggregate connexin43, β-Catenin, desmosomale Strukturen sowie N-Cadherin nachgewiesen werden. Diese Zellverbindungsstrukturen, die über das gesamte Aggregate gleichmäßig verteilt sind, zeigen, dass es sich bei dem Herzzellaggregat um ein funktionierendes Synzytium handelt. Es kann daher von einem in sich geschlossenen und spontan funktionierenden Herzzellaggregat gesprochen werden, in dem Herzmuskelzellen mit anderen Herzmuskelzellen elektrisch gekoppelt sind. Weiterhin sind aufgrund der spontanen und gleichmäßigen Kontraktion Herzschrittmacherzellen im Aggregat vorhanden.

Speziell für die Anwendung ist das Synzytium bedeutend, da auf Zellverband-Ebene und nicht nur auf Einzelzell-Ebene Untersuchungen angestrebt werden können. Somit kann der Ursache-Wirkungs-Komplex intensiver untersucht werden.

### Erläuterung von Fig. 7:

PCR: elfa - housekeeping gene, a-actin-alpha actin; myh6 - cardiac myosin heavy chain 6; cx43 - connexion 43. SCC1-SCC3 - Proben von Herzzellaggregaten, PK -positiv Kontrolle (Herz einer adulten Regenbogenforelle); NK - negative Kontrolle - non-kardiale Zellen aus P3 von einer Regenbogenforellenlarve

### Elektronenmikroskopische Aufnahmen:

Neben der gestreiften Muskulatur und Unterteilung in Sarkomere ist ein weiteres Indiz für die Bildung von komplexen Herzzellaggregaten und nicht einfachen Kardiomyozyten der Einbau von intercalated disks, welche den junctional Komplex repräsentieren. Dieser Komplex ist für die Zellverbindung verantwortlich. In den Aggregaten konnten diverse *gap junctions* festgestellt werden. Weiterhin wurden auch T-Tubuli erfasst, die, wie auch in humanen Kardiomyozyten, auf Höhe von Z-Banden liegen und zudem meist mit einer sarkoplasmatischem Retikulum-Zisterne assoziiert sind. Herzmuskelzellen besitzen zudem eine hohe Anzahl von Mitochondrien im Zytoplasma, um den aeroben Metabolismus, der für das Herz entscheidend ist, zu gewährleisten. Auch in den generierten Aggregaten konnten Mitochondrien per Elektronenmikroskopie nachgewiesen werden.

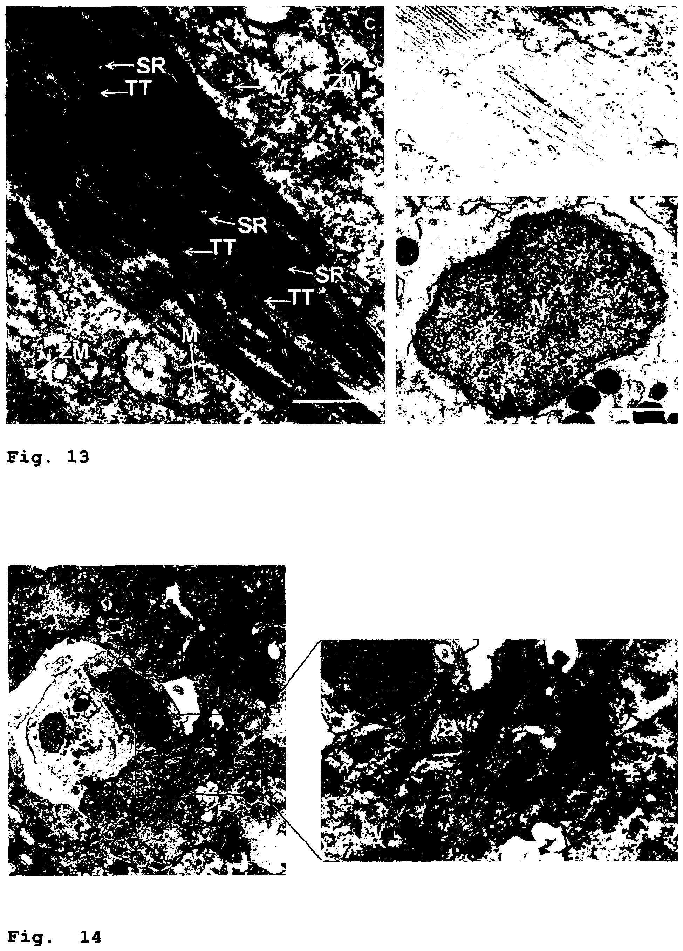

Fig. 13 zeigt einen longitudinalen Schnitt durch ein Herzzellaggregat (Elektronenmikroskopische Aufnahme). Die transversal orientierenden Abschnitte der intercalated disk bestehen aus *fascia adhaerens* und diversen Desmosomen (nachgewiesen mit der Immunfluoreszenz) (a, b). Sarkomere mit sarkoplasmatischen Retikulum (SR) und T-Tubulus (TT) in den Z-Scheiben sowie Mitochondrien (M). Sarkoplasmatisches Retikulum (SR) mit T-Tubuls (TT) vereint (d). Nachweis von Nuclei (N) (e) . Skala: 1 µm.

Auch für Anwendungen in der Entwicklungsbiologie sind die generierten Aggregate von großem Interesse, da hier auf Zellebene Vorgänge untersucht werden können. So wurde mittels EM der Nachweis erbracht, dass die Kardiomyozyten mittels Zell-Zell-Kommunikation entstehen. Aufnahmen nicht vollständig ausgebildete Kardiomyozyten wiesen am Zellrand nur vereinzelte Muskelfilamente auf, so dass der Anschein einer beginnenden Differenzierung zur Kardiomyozyte erweckt wurde. Weiterhin wurden bei diesen Zellen auch in den Nachbarzellen beginnende Querstreifungen ersichtlich, die ebenfalls am Zellrand zu den Nachbarzellen lagen. In diesen Fällen besteht der Eindruck einer Zell-Zell-Kommunikation für die Generierung zur Herzzelle.

Fig. 14 zeigt die Entstehung einer Herzelle. Erste Sarkomere sind an benachbarten Zellen erkennbar.

### BEISPIEL 5

### Elektrophysiologie der Zellaggregate

Für die Verwendung der Zellaggregate als Testsystem in der Pharmakologie ist die Elektrophysiologie wichtig. Die Messung der elektrischen Aktivität erfolgte mittels extrazellulärer Messung via MEA (Multielektronenarray) sowie intrazellulärer Messung via scharfer Elektrode.

### 3.1. Extrazelluläre Ableitung

Die extrazelluläre Signalableitung von kardialen Zellen mit Hilfe eines Multielektrodenarrays (MEA) erlaubt die simultane Detektion der Aktivität von mehreren Stellen eines Zellnetzwerkes.

Die Untersuchungen mit dem MEA bewiesen, dass die Zellen des Aggregates miteinander arbeiten, wie es auch im Herz von allen Vertebraten der Fall ist, und im gesamten Aggregat Aktionspotentiale messbar sind. Die Weiterleitungseigenschaft bestätigt den Nachweis eines funktionierenden Zellverbundes.

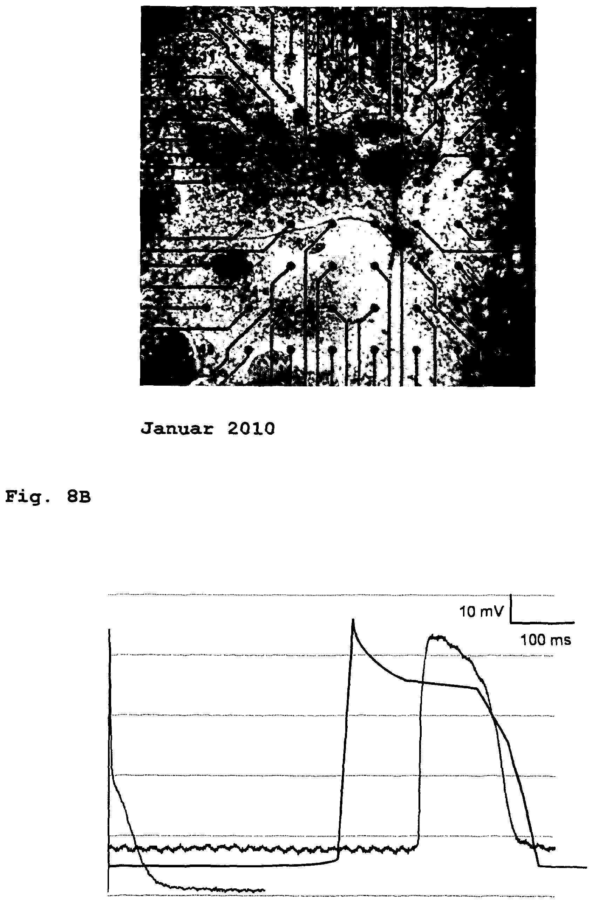

Aus Fig. 8 ist ersichtlich, dass die Aggregate zum einen durch Zellvermehrung wachsen, so dass sie mehr Elektroden belegen und ihr Feldpotential ausweiten. Weiterhin verändern sie auch ihre Position auf dem MEA. Das kleine Aggregat, welches in Fig. 8A unten lag, wanderte in Richtung des oberen und größeren Aggregates. Am Ende haben sich beide Aggregate aus zwei unterschiedlichen Larven vereinigt (Fig. 8B), so dass aus ehemals zwei unterschiedlichen Kontraktionsfrequenzen ein kontrahierendes Aggregat wurde. Zellverbindungsproteine sind dafür nötig, die in diesem Fall aufgebaut wurden.

Diese Besonderheit, dass zwei Zellen aus unterschiedlichen Organismen sich vereinen, ist bei der Transplantationsmedizin Voraussetzung. So könnten an diesen Herzzellaggregaten die Phänomene der Abstoßung besser untersucht werden.

### 3.2. Intrazelluläre Ableitung

Im Gegensatz zu der extrazellulären Ableitung können mit intrazellulären Ableitungen transmembrane Spannungen gemessen werden. Dadurch können die Mechanismen der Potentialerzeugung, das Membranpotential sowie die beteiligten Ionenströme direkt gemessen werden.

Mittels scharfer Elektrode wurden die Eigenschaften von Aktionspotentialen sowie die Untersuchung auf bestimmte Kanäle (Kalium- und dem Calciumkanal) vorgenommen.

Es konnte bewiesen werden, dass die Aktionspotentialparameter der Aggregate dem der Menschen sehr ähnlich sind. Besonders charakteristisch für humane Kardiomyozyten ist die ausgeprägte Plateauphase, die das generierte Aggregat ebenfalls aufweist (Fig. 9). Im Gegensatz dazu wird auch demonstriert, dass Mauszellen, die bisher hauptsächlich als Testmodelle verwendet werden, in ihrem elektrophysiologischen Potentials sehr von humanen APs abweichen und daher ungeeignet sind. Untersuchungen der Aggregaten offenbarten auch die Korrelation zwischen der Aktionspotentialdauer und der Periode, die auch bei höheren (Mensch), aber nicht bei niederen Säugetieren (z.B. Maus) vorkommt.

### 3.3. Medikamententests

Für die Funktion als Testmodell ist die Reaktion der Aggregate auf Medikamente, die bestimmte Kanäle im Herzen beeinflussen von Bedeutung. Es wurde gezeigt, dass die Aggregate auf bekannte Herzmedikamente in derselben Art und Weise reagieren, wie das humane Herz. Untersuchungen fanden mit Isoproterenol (Calciumkanalagonist) Fig. 10A) und Rilmakalim (Kaliumkanalblocker) (Fig. 10B) statt. Es konnte beobachtet werden, dass sich die Aggregate nach der Medikamententestung durch Mediumwechsel erholen und für erneute Versuche zur Verfügung stehen.

## Patentansprüche

1. *In vitro*-Verfahren zur Erzeugung von kontrahierenden Fischzellaggregaten, umfassend die Schritte:
a) Mechanische Zerkleinerung und/oder partielle enzymatische Verdauung von Fischembryonen oder Fischlarven im Entwicklungsstadium von Anfang Augenpunktstadium bis Ende Dottersackstadium;
b) Überführung des zerkleinerten und/oder partiell verdauten Fischgewebes in enzymfreies Medium und Abzentrifugation des Überstands zur Gewinnung eines Zellpellets;
c) Resuspension des Zellpellets in Zellkulturmedium;
d) Kultivierung der Zellen unter mindestens einmaligem Mediumwechsel bis zur Bildung von spontan kontrahierenden Zellaggregaten.

2. Verfahren nach Anspruch 1, **dadurch gekennzeichnet, dass**
die enzymatische Verdauung eine Trypsinbehandlung umfasst.

3. Verfahren nach Anspruch 2, **dadurch gekennzeichnet, dass**
die Trypsinbehandlung in einem Puffer mit 0.01 - 1 % Trypsin für einen Zeitraum von 30 Sekunden bis 2 Minuten erfolgt.

4. Verfahren nach einem der Ansprüche 1-3, **dadurch gekennzeichnet, dass**
die Kultivierung der Zellen in Schritt d) für einen Zeitraum von mindestens 3 bis 7 Tagen erfolgt.

5. Verfahren nach einem der Ansprüche 1-4, **dadurch gekennzeichnet, dass** die Zellkultur mehrfach passagiert wird.

6. Verfahren nach einem der Ansprüche 1-5, **dadurch gekennzeichnet, dass** die Kultivierung bei einer Temperatur von 4-28 °C und einem CO₂-Gehalt von 1- 5 % erfolgt.

7. Verfahren nach einem der Ansprüche 1-6, **dadurch gekennzeichnet, dass** die Fischembryonen oder Fischlarven aus Spezies der Teleostei ausgewählt sind.

8. Verfahren nach Anspruch 7, **dadurch gekennzeichnet, dass**
die Fischembryonen oder Fischlarven aus Spezies der Heringsfische (Clupeoidei), Lachsartigen (Salmonoidei), der Karpfenfische (Cyprinidae), Aale (Anguillidae), Barsche (Percidae), Dorsche (Gadidae), Welse (Siluroidae), Plattfische (Pleuronectiformes), Hornhechte (Beloniformes) und Störe (Acipenseriformes) ausgewählt sind.

9. Verfahren nach Anspruch 8, **dadurch gekennzeichnet, dass**
die Fischembryonen oder Fischlarven aus Spezies der Lachsartigen (Salmonoidei) ausgewählt sind.

10. Verfahren nach Anspruch 9, **dadurch gekennzeichnet, dass**
die Fischembryonen oder Fischlarven von Lachsen, Forellen, insbesondere Bachforelle, Regenbogenforelle, Saiblingen, Huchen, Felchen, Renken, Äschen oder Stinten stammen.

11. Verfahren nach einem der Ansprüche 1-10, **dadurch gekennzeichnet, dass** in Schritt a) Fischlarven kurz nach dem Schlupf verwendet werden.

12. Materialzusammensetzung, umfassend spontan kontrahierende Fischzellaggregate, die mit dem Verfahren nach einem der Ansprüche 1-11 erhältlich sind.

13. Materialzusammensetzung nach Anspruch 11, umfassend ferner ein Träger- oder Hüllmaterial.

14. Zellkultur, umfassend spontan kontrahierende Fischzellaggregate.

15. Zellkultur nach Anspruch 14, welche für mindestens 1 Passage stabil ist.

16. Materialzusammensetzung nach Anspruch 12 oder 13 oder Zellkultur nach Anspruch 14 oder 15, umfassend ballförmige oder röhrenförmige kontrahierende Zellaggregate.

17. Materialzusammensetzung oder Zellkultur nach Anspruch 12 oder 14, umfassend mindestens ein spontan kontrahierendes Fischzellaggregat, welches durch Zusammenwachsen von Zellaggregaten von zwei oder mehr verschiedenen Fischlarven entstanden ist und damit Zellmaterial von mindestens zwei verschiedenen Fischlarven umfasst.

18. Verwendung der Materialzusammensetzung oder der Zellkultur nach einem der Ansprüche 12-17 zum Testen von biologischen Wirkstoffen oder Pharmazeutika.

19. Verwendung nach Anspruch 18, **dadurch gekennzeichnet, dass** die biologischen Wirkstoffe oder Pharmazeutika hinsichtlich ihrer Wirkung auf Herzmuskelzellen oder andere Herzzellen getestet werden.

20. Materialzusammensetzung oder Zellkultur nach einem der Ansprüche 12-17 zur medizinischen Verwendung in der Regeneration von Herzzellaggregaten.

21. *In* vitro-System zum Testen von biologischen Wirkstoffen oder Pharmazeutika hinsichtlich ihrer Wirkung auf Herzmuskelzellen oder andere Herzzellen, umfassend eine Materialzusammensetzung oder Zellkultur nach einem der Ansprüche 12-17.

## Claims

1. An *in vitro* method for producing contracting fish cell aggregates, comprising the steps:
a) mechanical comminution and/or partial enzymatic digestion of fish embryos or fish larvae in the development stage from the beginning of the eyed egg stage to the end of the yolk sac stage;
b) transfer of the comminuted and/or partially digested fish tissue into an enzyme-free medium and removing the supernatant by centrifuging to obtain a cell pellet;
c) resuspension of the cell pellet in a cell culture medium;
d) cultivation of the cells, with the medium being changed at least once, until spontaneously contracting cell aggregates form.

2. The method according to claim 1, **characterized in that**
the enzymatic digestion comprises a trypsin treatment.

3. The method according to claim 2, **characterized in that**
the trypsin treatment is performed in a buffer with 0.01-1% trypsin for a period of 30 seconds to 2 minutes.

4. The method according to any one of claims 1-3, **characterized in that**
the cultivation of the cells in step d) is carried out for a period of at least 3 to 7 days.

5. The method according to any one of claims 1-4, **characterized in that** the cell culture is passaged several times.

6. The method according to any one of claims 1-5, **characterized in that** the cultivation is carried out at a temperature of 4-28°C and a CO₂ content of 1-5%.

7. The method according to any one of claims 1-6, **characterized in that** the fish embryos or fish larvae are selected from species of Teleostei.

8. The method according to claim 7, **characterized in that**
the fish embryos or fish larvae are selected from species of herrings (Clupeoidei), salmon-like fish (Salmonoidei), carps (Cyprinidae), eels (Anguillidae), perches (Percidae), cods (Gadidae), catfish (Siluroidae), flatfish (Pleuronectiformes), garfish (Beloniformes) and sturgeons (Acipenseriformes).

9. The method according to claim 8, **characterized in that**
the fish embryos or fish larvae are selected from species of salmon-like fish (Salmonoidei).

10. The method according to claim 9, **characterized in that**
the fish embryos or fish larvae are derived from salmons, trouts, in particular brown trouts, rainbow trouts, samlets, huchens, whitefish, graylings or smelts.

11. The method according to any one of claims 1-10, **characterized in that** in step a), fish larvae are used shortly after the hatching.

12. A material composition, comprising spontaneously contracting fish cell aggregates which are obtainable by the method according to any one of claims 1-11.

13. The material composition according to claim 11, further comprising a carrier or envelope material.

14. A cell culture, comprising spontaneously contracting fish cell aggregates.

15. The cell culture according to claim 14 which is stable for at least 1 passage.

16. The material composition according to claim 12 or claim 13 or the cell culture according to claim 14 or claim 15, comprising spherical or tubular contracting cell aggregates.

17. The material composition or the cell culture according to claim 12 or claim 14, comprising at least one spontaneously contracting fish cell aggregate which is formed by the coalescence of cell aggregates from two or more different fish larvae and, thus, comprises cell material of at last 2 different fish larvae.

18. Use of the material composition or the cell culture according to any one of claims 12-17 for testing biological active substances or pharmaceuticals.

19. The use according to claim 18, **characterized in that** the biological active substances or pharmaceuticals are tested with regard to their effect on heart muscle cells or other heart cells.

20. The material composition or the cell culture according to any one of claims 12-17 for medical use in the regeneration of heart cell aggregates.

21. An *in vitro* system for testing biological active substances or pharmaceuticals with regard to their effect on heart muscle cells or other heart cells, comprising a material composition or a cell culture according to any one of claims 12-17.

## Revendications

1. Procédé *in vitro* pour la production d'agrégats de cellules de poisson se contractant, comprenant les étapes de :
a) réduction mécanique et/ou digestion enzymatique partielle d'embryons de poisson ou de larves de poisson au stade de développement du début du stade de fécondation à la fin du stade de l'alevin ;
b) transfert du tissu de poisson réduit et/ou partiellement digéré dans un milieu sans enzyme et centrifugation du résidu pour obtenir un amas cellulaire ;
c) remise en suspension de l'amas cellulaire dans un milieu de culture cellulaire ;
d) culture des cellules avec au moins un changement de milieu jusqu'à la formation d'agrégats cellulaires se contractant spontanément.

2. Procédé selon la revendication 1, **caractérisé en ce que** la digestion enzymatique comprend un traitement à la trypsine.

3. Procédé selon la revendication 2, **caractérisé en ce que** le traitement à la trypsine s'effectue dans un tampon avec 0,01 à 1 % de trypsine pendant une durée de 30 secondes à 2 minutes.

4. Procédé selon l'une des revendications 1 à 3, **caractérisé en ce que**
la culture des cellules s'effectue à l'étape d) pendant une durée d'au moins 3 à 7 jours.

5. Procédé selon l'une des revendications 1 à 4, **caractérisé en ce que** la culture cellulaire est soumise à plusieurs passages.

6. Procédé selon l'une des revendications 1 à 5, **caractérisé en ce que** la culture s'effectue à une température de 4 à 28 °C et à une teneur en CO₂ de 1 à 5 %.

7. Procédé selon l'une des revendications 1 à 6, **caractérisé en ce que** les embryons de poisson ou les larves de poisson sont choisis parmi les espèces de téléostéens.

8. Procédé selon la revendication 7, **caractérisé en ce que** les embryons de poisson ou les larves de poisson sont choisis parmi les espèces de harengs (clupéidés), de saumons (salmonidés), de carpes (cyprinidés), d'anguilles (anguillidés), de perches (percidés), de morues (gadidés), de silures (siluridés), de poissons plats (pleuronectiformes), d'orphies (béloniformes) et d'esturgeons (acipensériformes).

9. Procédé selon la revendication 8, **caractérisé en ce que** les embryons de poisson ou les larves de poisson sont choisis parmi les espèces de saumons (salmonidés).

10. Procédé selon la revendication 9, **caractérisé en ce que** les embryons de poisson ou les larves de poisson proviennent de saumons, de truites, en particulier de truites brunes, de truites arc-en-ciel, d'ombles, de huchons, de féras, de corégones, d'ombres ou d'éperlans.

11. Procédé selon l'une des revendications 1 à 10, **caractérisé en ce que** à l'étape a), des larves de poissons sont utilisées peu après l'éclosion.

12. Composition de matériau, comprenant des agrégats de cellules de poisson se contractant spontanément, qui peuvent être obtenus avec le procédé selon l'une des revendications 1 à 11.

13. Composition de matériau selon la revendication 11, comprenant en outre un matériau de support ou d'enveloppe.

14. Culture cellulaire, comprenant des agrégats de cellules de poisson se contractant spontanément.

15. Culture cellulaire selon la revendication 14, qui est stable pour au moins 1 passage.

16. Composition de matériau selon la revendication 12 ou 13 ou culture cellulaire selon la revendication 14 ou 15, comprenant des agrégats de cellules se contractant sous forme de balle ou sous forme de tube.

17. Composition de matériau ou culture cellulaire selon la revendication 12 ou 14, comprenant au moins un agrégat de cellules de poisson se contractant spontanément, qui s'est formé par fusion d'agrégats cellulaire de deux ou plusieurs larves de poisson différentes et comprend ainsi un matériau cellulaire d'au moins deux larves de poisson différentes.

18. Utilisation de la composition de matériau ou de la culture cellulaire selon l'une des revendications 12 à 17 pour tester des substances actives biologiques ou des produits pharmaceutiques.

19. Utilisation selon la revendication 18, **caractérisée en ce que** les substances actives biologiques ou les produits pharmaceutiques sont testés en ce qui concerne leur effet sur des cellules du muscle cardiaque ou d'autres cellules cardiaques.

20. Composition de matériau ou de culture cellulaire selon l'une des revendications 12 à 17 pour l'utilisation médicale dans la régénération d'agrégats de cellules cardiaques.

21. Système de test in vitro de substances actives biologiques ou de produits pharmaceutiques en ce qui concerne leur effet sur des cellules du muscle cardiaque ou d'autres cellules cardiaques, comprenant une composition de matériau ou une culture cellulaire selon l'une des revendications 12 à 17.
